# EUROPÄISCHE PATENTSCHRIFT

(11) **EP 2 007 444 B1**
(45) Veröffentlichungstag und Bekanntmachung des Hinweises auf die Patenterteilung: **20.10.2010**
(21) Anmeldenummer: 07723744.4
(22) Anmeldetag: 29.03.2007
(51) Int. Cl.: A61L 15/26, A61L 15/42

(54) **POLYURETHAN-SCHÄUME FÜR DIE WUNDBEHANDLUNG**
POLYURETHANE FOAMS FOR TREATING WOUNDS
MOUSSES POLYURÉTHANE POUR SOIGNER DES PLAIES

(30) Priorität: 08.04.2006 DE 102006016636
(43) Veröffentlichungstag der Anmeldung: 31.12.2008
(73) Patentinhaber: Bayer MaterialScience AG, 51368 Leverkusen (DE); Bayer Innovation GmbH, 40225 Düsseldorf (DE)
(72) Erfinder: RISCHE, Thorsten, 59423 Unna (DE); MAGER, Michael, 51375 Leverkusen (DE); HECKES, Michael, 47800 Krefeld (DE); RUDHARDT, Daniel, 50823 Köln (DE); GERTZMANN, Rolf, 51375 Leverkusen (DE); DIETZE, Melita, 40699 Erkrath (DE); FUGMANN, Burkhard, 40878 Ratingen (DE)
(86) Internationale Anmeldenummer: PCT/EP2007/002800
(87) Internationale Veröffentlichungsnummer: WO 2007/115696

(56) Entgegenhaltungen:
- WO-A-2004/037307
- WO-A-2007/115781
- DE-A1- 2 264 853
- US-A- 5 914 125
- US-A1- 2004 210 026
- US-B1- 6 605 666

## Beschreibung

Die Erfindung betrifft ein Verfahren zur Herstellung von Polyurethan-Schäumen für die Wundbehandlung, bei welchem eine Zusammensetzung, enthaltend eine Polyurethan-Dispersion und spezielle Koagulantien, aufgeschäumt und getrocknet wird.

Die Verwendung von Wundauflagen aus Schäumen zur Behandlung von nässenden Wunden ist Stand der Technik. Aufgrund ihres hohen Absorptionsvermögens und ihrer guten mechanischen Eigenschaften werden in Regel Polyurethan-Schäume eingesetzt, welche durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-) Additiven hergestellt werden. In der Regel werden hierbei aromatische Diisocyanate eingesetzt, da sich diese am besten Verschäumen lassen. Zahlreiche Ausführungsformen dieser Verfahren sind bekannt, beispielsweise beschrieben in US 3,978,266, US 3,975,567, US 5,914,125 und EP 0 059 048. Die vorgenannten Verfahren weisen jedoch den Nachteil auf, dass reaktive Mischungen eingesetzt werden müssen, enthaltend Diisocyanate oder entsprechende NCO-funktionelle Prepolymere, deren Handhabung technisch aufwendig ist, da z.B. entsprechende Schutzmaßnahmen erforderlich sind.

Eine Alternative zum vorstehend beschriebenen Verfahren, in welchem Diisocyanate bzw. NCO-funktionelle Polyurethan-Prepolymere eingesetzt werden, ist ein Verfahren basierend auf Polyurethan-Dispersionen (die im wesentlichen frei von Isocyanat-Gruppen sind), in welche man in Gegenwart geeigneter (Schaum-) Additive durch kräftiges Rühren Luft einträgt. Nach dem Trocknen und Aushärten werden so genannte mechanische Polyurethan-Schäume erhalten. Solche Schäume sind im Zusammenhang mit Wundauflagen beschrieben in EP 0 235 949 und EP 0 246 723, wobei dem Schaum entweder ein selbsthaftendes Polymer zugesetzt oder der Schaum auf einen Film eines selbsthaftenden Polymers aufgebracht wird. Die in EP 0 235 949 und EP 0 246 723 aufgeführten Beispiele beschreiben darüber hinaus zwingend die Verwendung von Polyaziridinen als Vernetzer, was jedoch nach heutigem Stand des Wissens hinsichtlich deren Toxizität nicht mehr akzeptabel ist. Zur Vernetzung müssen überdies hohe Einbrenntemperaturen angewandt werden, angegeben sind 100°C bis 170°C. In US 4,655,210 ist die Verwendung der vorgenannten mechanischen Schäume für Wundauflagen beschrieben, die einen speziellen Aufbau aus Träger, Schaum und Hautkontakt-Schicht aufweisen. Die nach den EP 0 235 949 und EP 0 246 723 beschriebenen Verfahren hergestellten Schäume weisen darüber hinaus den großen Nachteil auf, dass die erhaltenen Schäume nur in geringem Maße offenzellig sind, wodurch die Absorption von physiologischer Salzlösung sowie auch die Wasserdampfdurchlässigkeit gering sind.

Zur Versorgung von Wunden mit komplexer Topologie oder zur Bedeckung besonders tiefer Wunden ist die Verwendung gebrauchsfertiger, industriell hergestellter flächiger Wundauflagen sehr schwierig, da eine optimale Bedeckung der Wundoberfläche in der Regel nicht gelingt, wodurch der Heilungsprozess verzögert wird. Um eine bessere Bedeckung tiefer Wunden zu erreichen, wurde vorgeschlagen, Granulate aus mikroporösen Polyurethanen anstelle kompakter Wundauflagen einzusetzen (EP-A-0 171 268). Jedoch wird auch hierdurch keine optimale Bedeckung der Wunde erreicht.

Der Auftrag einer (fließfähigen) Zusammensetzung, welche sich der Wundform optimal anpasst, würde die Nachteile flächiger Wundauflagen beseitigen. Die beiden vorstehend beschriebenen Verfahren, in welchen zur Herstellung der Polyurethan-Schäume entweder Diisocyanate bzw. NCO-funktionelle Polyurethan-Prepolymere oder Polyurethan-Dispersionen in Kombination mit Polyaziridinen eingesetzt werden, sind hierzu jedoch unbrauchbar: reaktive Zusammensetzungen, die freie Isocyanat-Gruppen enthalten, können nicht direkt auf die Haut aufgetragen werden, auch wenn dies verschiedentlich vorgeschlagen wurde (siehe beispielsweise WO 02/26848). Aber auch die Verwendung von Polyurethan-Dispersionen mit Polyaziridinen als Vernetzer ist aufgrund der nach heutigem Stand des Wissens toxikologisch bedenklichen Eigenschaften des Vernetzer ausgeschlossen.

Aufgabe der vorliegenden Erfindung war daher die Bereitstellung von Polyurethan-Schäumen für die Wundbehandlung, wobei die Herstellung unter Verwendung einer Zusammensetzung erfolgt, die frei von Isocyanat-Gruppen ist. Die Herstellung des Polyurethan-Schaums soll grundsätzlich auch bei Umgebungsbedingungen durchgeführt werden können, wobei die gebildeten Polyurethan-Schäume neben guten mechanischen Eigenschaften, eine hohe Absorption von physiologischer Salzlösung und eine hohe Wasserdampfdurchlässigkeit aufweisen sollen. Hierzu ist erforderlich, dass der Polyurethan-Schaum eine gewisse Offenzelligkeit aufweist. Die Zusammensetzung soll darüber hinaus zum direkten Auftrag auf die Haut geeignet sein, z.B. durch Sprühen oder Gießen, damit die Wunde optimal mit dem Polyurethan-Schaum bedeckt wird; eine schnelle Trocknung ist hierzu wesentlich.

Es wurde nun gefunden, dass man aus Zusammensetzungen, enthaltend Polyurethan-Dispersionen und spezielle kationische Koagulantien, beide frei von Isocyanat-Gruppen, bereits bei Umgebungsbedingungen Polyurethan-Schäume herstellen kann, die gute mechanische Eigenschaften, eine hohe Absorption von physiologischer Salzlösung und eine hohe Wasserdampfdurchlässigkeit aufweisen. Die Polyurethan-Schäume zeigen zumindest teilweise eine offenzellige Porenstruktur. Die fließfähigen Zusammensetzungen können überdies durch Sprühen oder Gießen direkt auf die Haut aufgebracht werden.

Gegenstand der vorliegenden Erfindung ist daher ein Verfahren zur Herstellung von Wundauflagen aus Polyurethan-Schäumen, bei dem eine Zusammensetzung enthaltend eine wässrige, anionisch hydrophilierte Polyurethan-Dispersionen (I) und ein kationisches Koagulans (II), aufgeschäumt und getrocknet wird.

Wundauflagen aus Polyurethan-Schäumen im Sinne der Erfindung sind poröse Materialien, bevorzugt mit zumindest teilweise vorhandener Offenzelligkeit, welche im wesentlichen aus Polyurethanen bestehen und Wunden im Sinne einer sterilen Abdeckung vor Keimen bzw. Umgebungseinflüssen schützen, eine schnelle und hohe Absorption von physiologischer Salzlösung bzw. Wundflüssigkeit aufweisen, durch geeignete Feuchtdurchlässigkeit für ein geeignetes Wundklima sorgen und eine ausreichende mechanische Festigkeit aufweisen.

Die in den erfindungswesentlichen Zusammensetzungen enthaltenen wässrigen, anionisch hydrophilierten Polyurethan-Dispersionen (I) sind erhältlich, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol und
   A4) gegebenenfalls isocyanatreaktiven, anionischen oder potentiell anionischen und/oder gegebenenfalls nichtionischen Hydrophilierungsmitteln,
   hergestellt werden,
B) dessen freie NCO-Gruppen dann ganz oder teilweise
   B1) gegebenenfalls mit aminofunktionellen Verbindungen mit Molekulargewichten von 32 bis 400 g/mol und
   B2) mit isocyanatreaktiven, bevorzugt aminofunktionellen, anionischen oder potentiell anionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

Um eine anionische Hydrophilierung zu erreichen müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO⁻ oder -SO₃⁻ oder -PO₃²⁻ als anionische bzw. deren ganz oder teilweise protonierte Säureformen als potentiell anionische Gruppen aufweisen.

Bevorzugte wässrige, anionische Polyurethan-Dispersionen (I) haben einen niedrigen Grad an hydrophilen anionischen Gruppen, bevorzugt von 0,1 bis 15 Milliequivalenten pro 100 g Festharz.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers 1,05 bis 3,5, bevorzugt 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 und 125 %, besonders bevorzugt zwischen 60 und 120 % beträgt.

Geeignete Polyisocyanate der Komponente A1) sind die dem Fachmann an sich bekannten aromatischen, araliphatischen, aliphatischen oder cycloaliphatischen Polyisocyanate einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit C₁-C₈-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie z.B. 4-Isocyanatomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbemsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Die Hydroxylgruppen aufweisenden Polycarbonate sind bevorzugt linear gebaut.

Ebenfalls können in A2) Polyetherpolyole eingesetzt werden.

Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrins an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung 20 bis 80 Gew.-% und der Anteil an Polytetramethylenglykolpolyolen 80 bis 20 Gew.-% beträgt. Bevorzugt ist ein Anteil von 30 bis 75 Gew.-% an Polytetramethylenglykolpolyolen und ein Anteil von 25 bis 70 Gew.-% an Polycarbonatpolyolen. Besonders bevorzugt ist ein Anteil von 35 bis 70 Gew.-% an Polytetramethylenglykolpolyolen und ein Anteil von 30 bis 65 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2) mindestens 50 Gew.-%, bevorzugt 60 Gew.-% und besonders bevorzugt mindestens 70 Gew.-% beträgt.

Die Verbindungen der Komponente A3) besitzen Molekulargewichte von 62 bis 400 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-e-hydroxycapronsäureester, ω-Hydroxyhexyl-γ-hydroxybuttersäure-ester, Adipinsäure-(β-hydroxyethyl)ester oder Terephthalsäurebis(β-hydroxyethyl)-ester.

Ferner können in A3) auch monofunktionelle, isocyanatreaktive, Hydroxylgruppen-haltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente A3) sind 1,6-Hexandiol. 1,4-Butandiol, Neopentylglykol und Trimethylolpropan.

Unter anionisch bzw. potentiell anionisch hydrophilierenden Verbindungen der Komponente A4) werden sämtliche Verbindungen verstanden, die mindestens eine isocyanatreaktive Gruppe wie eine Hydroxylgruppe aufweisen sowie mindestens eine Funktionalität wie z.B. -COO-M⁺, -SO₃⁻ M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5-9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente A4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die 40 bis 100 mol-% Ethylenoxid- und 0 bis 60 mol-% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin.

Unter anionisch bzw. potentiell anionisch hydrophilierenden Verbindungen der Komponente B2) werden sämtliche Verbindungen verstanden, die mindestens eine isocyanatreaktive Gruppe, bevorzugt eine Amino-Gruppe aufweisen, sowie mindestens eine Funktionalität wie z.B. -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiaminpropyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carobonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten Komponente A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,1 bis 15 Gew.-% Summe der Komponenten Komponente A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z.B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet. Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen 1,05 bis 3,5, bevorzugt 1,2 bis 3,0, besonders bevorzugt 1,3 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldüsopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (I) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen (I) beträgt bevorzugt 40 bis 70, besonders bevorzugt 50 bis 65, ganz besonders bevorzugt 55 bis 65 und insbesondere 60 bis 65 Gew.-%.

Als Koagulantien (II) können in den Zusammensetzungen alle mindestens 2 kationische Gruppen enthaltende organische Verbindungen, bevorzugt alle bekannten kationischen Flockulations- und Fällungsmittel des Stands der Technik, wie kationische Homo- oder Copolymere von Salzen des Poly[2-(N,N,N-trimethylamino)-ethylacrylats], Polyethylenimins, Poly[N-(dimethylaminomethyl)acrylamids], substituierter Acrylamide, substituierter Methacrylamide, N-Vinylformamids, N-Vinylacetamids, N-Vinylimidazols, 2-Vinylpyridins oder 4-Vinylpyridins eingesetzt werden.

Bevorzugte kationische Koagulanzien (II) sind Copolymere des Acrylamids, welche Struktureinheiten der allgemeinen Formel (2), besonders bevorzugt der allgemeinen Formeln (1) und (2) aufweisen 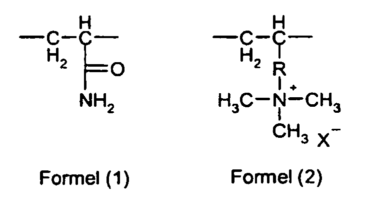 wobei
- R: C=O, -COO(CH₂)₂- oder -COO(CH₂)₃- und
- X⁻: ein Halogenidion, bevorzugt Chlorid ist

Bevorzugt weisen die Koagulantien (II) zahlenmittlere Molekulargewichte von 500.000 bis 50.000.000 g/mol auf.

Solche Koagulantien (II), auf Basis von Copolymeren des Acrylunids, werden beispielsweise unter dem Markennamen Praestol^{®} (Degussa Stockhausen, Krefeld, DE) als Flockungsmittel für Klärschlämme vertrieben. Bevorzugte Koagulantien vom Praestol^{®}-Typ sind Praestol^{®} K111L, K122L, K133L, BC 270L, K 144L, K 166L, BC 55L, 185K, 187K, 190K, K222L, K232L, K233L, K234L, K255L, K332L, K 333L, K 334L, E 125, E 150 sowie deren Mischungen. Ganz besonders bevorzugte Koagulationsmittel sind Praestol^{®} 185K, 187K und 190K sowie deren Mischungen.

Die Restgehalte an Monomeren, insbesondere Acrylat- und Acrylamidmonomere, liegen bei den Koagulantien vorzugsweise bei weniger als 1 Gew.-%, besonders bevorzugt bei weniger als 0,5 Gew.-% und ganz besonders bevorzugt bei weniger als 0,025 Gew.-%.

Die Koagulantien können in fester Form oder als wässrige Lösungen bzw. Dispersionen eingesetzt werden. Bevorzugt ist die Verwendung von wässrigen Dispersionen bzw. Lösungen.

Neben den Polyurethan-Dispersionen (I) und den Koagulantien (II) können auch Hilfs- und Zusatzstoffe (III) mitverwendet werden.

Beispiele für solche Hilfs- und Zusatzstoffe (III) sind Schaumhilfsmittel wie Schaumbildner und - stabilisatoren, Verdicker bzw. Thixotropiermittel, Antioxidantien, Lichtschutzmittel, Emulgatoren, Weichmacher, Pigmente, Füllstoffe und/oder Verlaufshilfsmittel.

Bevorzugt sind als Hilfs- und Zusatzstoffe (III) Schaumhilfsmittel wie Schaumbildner und - stabilisatoren enthalten. Geeignet sind beispielsweise handelsübliche Verbindungen wie Fettsäureamide, Sulfosuccinamide Kohlenwasserstoffsulfonate, -sulfate oder Fettsäuresalze, wobei der lipophile Rest bevorzugt 12 bis 24 Kohlenstoffatome enthält, sowie Alkylpolyglycoside, die nach dem Fachmann an sich bekannten Methoden durch Umsetzung von längerkettigen Monoalkoholen (4 bis 22 C-Atome im Alkylrest) mit Mono-, Di- oder Polysacchariden erhältlich sind (siehe z.B. Kirk-Othmer, Encyclopedia of Chemical Technology, John Wiley & Sons, Vol. 24, S. 29).

Bevorzugte Schaumhilfsmittel sind Sulfosuccinamide, Alkansulfonate oder -sulfate mit 12 bis 22 Kohlenstoffatomen im Kohlenwasserstoffrest, Alkylbenzosulfonate oder -sulfate mit 14 bis 24 Kohlenstoffatomen im Kohlenwasserstoffrest oder Fettsäureamide oder/und Fettsäuresalze mit 12 bis 24 Kohlenstoffatomen im Kohlenwasserstoffrest.

Solche Fettsäureamide sind vorzugsweise solche auf Basis von Mono- oder Di-(C₂-₃-alkanol)aminen. Die Fettsäuresalze können beispielsweise Alkalimetallsalze, Aminsalze oder unsubstituierte Ammoniumsalze sein.

Solche Fettsäurederivate basieren typischerweise auf Fettsäuren wie Laurinsäure, Myristinsäure, Palmitinsäure, Ölsäure, Stearinsäure, Ricinolsäure, Behensäure oder Arachidinsäure, Kokosfettsäure, Talgfettsäure, Sojafettsäure und deren Hydrierungsprodukten.

Besonders bevorzugte Schaumhilfsmittel sind Mischungen aus Sulfosuccinamiden und Ammoniumstearaten, wobei diese bevorzugt 20 bis 60 Gew.-% besonders bevorzugt 30 bis 50 Gew.-% an Ammoniumstearaten und bevorzugt 80 bis 40 Gew.-%, besonders bevorzugt 70 bis 50 Gew.-% an Sulfosuccinamiden enthalten.

Als Verdicker können handelsübliche Verdicker eingesetzt werden, wie Dextrin-, Stärke- oder Cellulosederivate wie Celluloseether oder Hydroxyethylcellulose, organische vollsynthetische Verdicker, auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren.

Grundsätzlich können, wenn auch nicht bevorzugt, die erfindungswesentlichen Zusammensetzungen auch Vernetzer wie unblockierte Polyisocyanate, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten.

Die erfindungswesentlichen Zusammensetzungen enthalten, bezogen auf Trockensubstanz, typischerweise 80 bis 99,5 Gewichtsteile der Dispersion (I), 0,5 bis 5 Gewichtsteile des kationischen Koagulans (II), 0 bis 10 Gewichtsteile Schaumhilfsmittel, 0 bis 10 Gewichtsteile Vernetzer und 0 bis 10 Gewichtsteile Verdicker.

Bevorzugt enthalten die erfindungswesentlichen Zusammensetzungen, bezogen auf Trockensubstanz, 85 bis 97 Gewichtsteile der Dispersion (I), 0,75 bis 4 Gewichtsteile des kationischen Koagulans (II), 0,5 bis 6 Gewichtsteile Schaumhilfsmittel, 0 bis 5 Gewichtsteile Vernetzer und 0 bis 5 Gewichtsteile Verdicker.

Besonders bevorzugt enthalten die erfindungswesentlichen Zusammensetzungen, bezogen auf Trockensubstanz, 89 bis 97 Gewichtsteile der Dispersion (I), 0,75 bis 3 Gewichtsteile des kationischen Koagulans (II), 0,5 bis 5 Gewichtsteile Schaumhilfsmittel, 0 bis 4 Gewichtsteile Vernetzer und 0 bis 4 Gewichtsteile Verdicker.

Neben den Komponenten (I), (II) und gegebenenfalls (III) können in den erfindungswesentlichen Zusammensetzungen auch andere wässrige Bindemittel eingesetzt werden. Solche wässrigen Bindemittel können z.B. aus Polyester-, Polyacrylat-, Polyepoxid- oder anderen Polyurethanpolymeren aufgebaut sein. Auch die Kombination mit strahlenhärtbaren Bindemitteln, wie sie z.B. in der EP-A-0 753 531 beschrieben sind, ist möglich. Ferner können auch andere anionische oder nichtionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Das Aufschäumen im erfindungsgemäßen Verfahren geschieht durch mechanisches Rühren der Zusammensetzung bei hohen Drehzahlen durch Schütteln oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der so erhaltene Schaum wird beim Aufschäumen oder unmittelbar danach auf ein Substrat aufgetragen oder in eine Form gegeben und getrocknet.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20°C beobachtet, so dass eine Trocknung auf verletztem menschlichen oder tierischen Gewebe problemlos möglich ist. Für eine schnellere Trocknung und Fixierung der Schäume werden jedoch bevorzugt Temperaturen oberhalb von 30°C benutzt. Bei der Trocknung sollten jedoch Temperaturen von 200°C bevorzugt 150°C, besonders bevorzugt 130°C nicht überschritten werden, da es anderenfalls u.a. zu unerwünschter Vergilbung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlem. Auch die Trocknung durch Führen des beschichteten Substrates über geheizte Oberflächen, z.B. Walzen, ist möglich.

Der Auftrag sowie die Trocknung können jeweils diskontinuierlich oder kontinuierlich durchgeführt werden, bevorzugt ist jedoch ein gänzlich kontinuierliches Verfahren

Als Substrate geeignet sind insbesondere Papiere oder Folien, die ein einfaches Ablösen der Wundauflage vor ihrem Einsatz zur Abdeckung einer verletzten Stelle ermöglichen. Ebenso kann als Substrat menschliches oder tierisches Gewebe wie Haut dienen, so dass ein direktes Verschließen einer verletzten Stelle durch eine in-situ hergestellte Wundauflage möglich ist.

Ein weiterer Gegenstand der vorliegenden Erfindung sind die nach dem erfindungsgemäßen Verfahren erhältlichen Wundauflagen.

Die Polyurethan-Schäume haben vor ihrer Trocknung typischerweise Schaumdichten von 50 bis 800 g/Liter, bevorzugt 100 bis 500 g/Liter, besonders bevorzugt 100 bis 250 g/Liter (Masse aller Einsatzstoffe [in g] bezogen auf das Schaumvolumen von einem Liter).

Die Polyurethan-Schäume besitzen nach ihrer Trocknung eine mikroporöse, zumindest teilweise offenporige Struktur mit miteinander kommunizierenden Zellen. Die Dichte der getrockneten Schäume liegt dabei typischerweise unterhalb von 0,4 g/cm³, bevorzugt ist sie geringer als 0,35 g/cm³, besonders bevorzugt 0,01 bis 0,3 g/cm³ und liegt ganz besonders bevorzugt bei 0,1 bis 0,3 g/cm³.

Das Absorptionsvermögen gegenüber physiologischer Salzlösung beträgt bei den Polyurethan-Schäumen typischerweise 100 bis 1500 %, bevorzugt 300 bis 1500 %, besonders bevorzugt 300 bis 800 % (Masse der aufgenommen Flüssigkeit bezogen auf die Masse des trockenen Schaums; Bestimmung nach DIN EN 13726-1, Teil 3.2). Die Durchlässigkeit gegenüber Wasserdampf beträgt typischerweise 2000 bis 8000 g/24 h * m², bevorzugt 3000 bis 8000 g/24 h * m², besonders bevorzugt 3000 bis 5000 g/24 h * m² (Bestimmung nach DIN EN 13726-2, Teil 3.2).

Die Polyurethan-Schäume weisen eine gute mechanische Festigkeit und hohe Elastizität auf. Typischerweise sind die Werte für die maximale Spannung größer als 0,2 N/mm² und die maximale Dehnung ist größer als 250 %. Bevorzugt ist die maximale Spannung größer als 0,4 N/mm² und die Dehnung größer als 350 % (Bestimmung nach DIN 53504).

Die Polyurethan-Schäume haben nach dem Trocknen typischerweise eine Dicke von 0,1 mm bis 50 mm, bevorzugt 0,5 mm bis 20 mm, besonders bevorzugt 1 bis 10 mm, ganz besonders bevorzugt 1 bis 5 mm.

Die Polyurethan-Schäume können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Sofern es zweckdienlich ist, kann im erfmdungsgemäßen Verfahren ein Schritt zur Sterilisation erfolgen. Ebenso ist es grundsätzlich möglich, die nach dem erfindungsgemäßen Verfahren erhältlichen Wundauflagen nach deren Herstellung zu Sterilisieren. Zur Sterilisation kommen die dem Fachmann an sich bekannten Verfahren zum Einsatz, bei denen eine Sterilisation durch thermische Behandlung geeignete Chemikalien wie Ethylenoxid oder Bestrahlung beispielsweise durch Gammabestrahlung erfolgt.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Aufgrund der breiten Einsetzbarkeit des erfindungsgemäßen Verfahrens und der darüber zugänglichen Wundauflagen ist es grundsätzlich möglich dieses in der industriellen Herstellung von Wundauflagen einzusetzen. Ebenso ist es aber auch möglich dieses zur Herstellung z.B. von Sprühpflastern einzusetzen, wobei die Wundauflage durch direkten Auftrag der Zusammensetzung auf eine Wunde und gleichzeitiges Aufschäumen und anschließendes Trocknen gebildet wird.

Zur industriellen Herstellung von Wundauflagen wird die Polyurethan-Dispersion (I) mit Schaumhilfsmitteln der vorstehend genannten Art vermischt, danach mechanisch durch Eintrag eines Gases wie Luft aufgeschäumt und schließlich durch Zugabe des Koagulans (II) koaguliert, wobei ein weiterhin verarbeitbarer, koagulierter Schaum erhalten wird. Dieser Schaum wird auf eine Unterlage aufgetragen und getrocknet. Aufgrund höherer Produktivität erfolgt die Trocknung typischerweise bei erhöhten Temperaturen von 30 bis 200°C, bevorzugt 50 bis 150°C, besonders bevorzugt 60 bis 130°C. Bevorzugt ist überdies eine mindestens zweistufige Trocknung beginnend bei Temperaturen von 40 bis 80°C und mit anschließender weiterer Trocknung bei erhöhten Temperaturen von 80 bis 140°C. Die Trocknung erfolgt in der Regel unter Verwendung von an sich bekannten Heiz- und Trockenapparaten, z.B. (Umluft-) Trockenschränken. Der Auftrag sowie die Trocknung können jeweils diskontinuierlich oder kontinuierlich durchgerührt werden, bevorzugt ist jedoch ein gänzlich kontinuierliches Verfahren.

Bei der Verwendung der erfindungswesentlichen Zusammensetzung bei der Herstellung eines Sprühpflasters werden die Polyurethan-Dispersion (I) und das Koagulans (II), welche jeweils gegebenenfalls Schaumhilfsmittel enthalten können, getrennt voneinander bereitgestellt und unmittelbar vor oder beim Auftrag auf das abzudeckende Gewebe miteinander gemischt. Das Aufschäumen geschieht hier durch gleichzeitige Entspannung eines Treibgases, welches in mindestens einer der Komponenten (I) und (II) enthalten war. Zur Verfestigung wird der gebildete Schaum anschließend getrocknet, wobei Temperaturen von 20 bis 40°C bereits ausreichen. Unter Zuhilfenahme zusätzlicher Wärmequellen wie Fön oder IR-Rotlichtlampe ist allerdings auch eine forcierte Wärmetrocknung bis maximal 80°C möglich.

Die dabei eingesetzten Treibmittel sind an sich aus der Polyurethanchemie bekannt. So sind beispielsweise geeignet n-Butan, i-Butan und Propan sowie Mischungen aus diesen Kohlenwasserstoffen, ebenso geeignet ist beispielsweise aber auch Dimethylether. Bevorzugt wird eine Mischung aus n-Butan, i-Butan und Propan eingesetzt, wodurch die gewünschten, feinzelligen Schäume erhalten werden. Das Treibmittel oder das Treibmittelgemisch wird dabei typischerweise in einer Menge von 1 bis 50 Gew.-%, bevorzugt 5 bis 40 Gew.-% und besonders bevorzugt 5 bis 20 Gew.-% eingesetzt, wobei die Summe aus eingesetzter Polyurethan-Dispersion (I), Koagulans (II). Treibmittel (-gemisch) sowie gegebenenfalls eingesetzter Hilfs- und Zusatzstoffe (III) 100 Gew.-% ergibt. Die Bereitstellung der Sprühpflaster erfolgt bevorzugt in Sprühdosen, wobei die Polyurethan-Dispersion (I) und das kationische Koagulans (II) getrennt voneinander enthalten sind und unmittelbar vor der Applikation miteinander vermischt werden. In einer oder in beiden Komponenten kann das Treibmittel enthalten sein. Eine oder beide der Komponenten (I) bzw. (II) können darüber hinaus gegebenenfalls noch Hilfs- und Zusatzstoffe (III) enthalten, bevorzugt Schaumhilfsmittel. Neben dem Sprühen ist auch Gießen der Zusammensetzung möglich.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

### Verwendete Substanzen und Abkürzungen:

| | |
|---|---|
| Diaminosulfonat: | NH₂-CH₂CH₂-NH-CH₂CH₂-SO₃Na (45 %ig in Wasser) |
| | |
| Desmophen^{®} C2200: | Polycarbonatpolyol, OH-Zahl 56 mg KOH/g, zahlenmittleres Molekulargewicht 2000 g/mol (BayerMaterialScience AG, Leverkusen, DE) |
| | |
| PolyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| | |
| PoIyTHF^{®} 1000: | Polytetramethylenglykolpolyol, OH-Zahl 112 mg. KOH/g, zahlenmittleres zahlenmittleres Molekulargewicht 1000 g/mol (BASF AG, Ludwigshafen, DE) |
| | |
| Polyether LB 25: | monofunktioneller Polyether auf Ethylenoxid-/Propylenoxidbasis, zahlenmittleres Molekulargewicht 2250 g/mol, OH-Zahl 25 mg KOH/g (BayerMaterialScience AG, Leverkusen, DE) |
| | |
| Stokal^{®} STA: | Schaumhilfsmittel auf Ammoniumstearat-Basis, Wirkstoffgehalt: 30 % (Bozzetto GmbH, Krefeld, DE) |
| | |
| Stokal^{®} SR: | Schaumhilfsmittel auf Succinamat-Basis, Wirkstoffgehalt: ca. 34 % (Bozzetto GmbH, Krefeld, DE) |
| | |
| Simulsol^{®} SL 26: | Alkylpolyglycosid auf Basis von Dodecylalkohol, ca. 52 %ig in Wasser, Seppic GmbH, Köln, DE |
| | |
| Praestol^{®} 185 K: | Kationisches Flockungshilfsmittel enthaltend die Strukturen der Formeln (1) und (2), Festkörpergehalt 25 % (Degussa AG, DE) |

Die Bestimmung der mittleren Teilchengrößen (angegeben ist das Zahlenmittel) der Polyurethan-Dispersionen (I) erfolgte mittels Laserkorrelations-Spektroskopie (Gerät: Malvern Zetasizer 1000, Malver Inst. Limited).

### Beispiel 1: Polyurethan-Dispersion 1

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht oder leicht unterschritten war. Das fertige Prepolymer wurde mit 4830 g Aceton gelöst und dabei auf 50°C abgekühlt und anschließend wurde eine Lösung aus 25,1 g Ethylendiamin, 116,5 g Isophorondiamin, 61,7 g Diaminosulfonat und 1030 g Wasser innerhalb von 10 min zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 61 % |
| Partikelgröße (LKS): | 312 nm |
| Viskosität (Viskosimeter, 23°C): | 241 mPas |
| pH (23°C): | 6,02 |

### Beispiel 2: Polyurethan-Dispersion 2

223,7 g PolyTHF^{®} 2000, 85,1 g PolyTHF^{®} 1000, 172,6 g Desmophen^{®} C2200 und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht oder leicht unterschritten war. Das fertige Prepolymer wurde mit 1005 g Aceton gelöst und dabei auf 50°C abgekühlt und anschließend wurde eine Lösung aus 5,70 Ethylendiamin, 26,4 g Isophorondiamin, 9,18 g Diaminosulfonat und 249,2 g Wasser innerhalb von 10 min zudosiert. Die Nachrührzeit betrug 10 min. Dann wurde durch Zugabe von 216 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 63% |
| Partikelgröße (LKS): | 495 nm |
| Viskosität (Viskosimeter, 23°C): | 133 mPas |
| pH (23°C): | 6,92 |

### Beispiel 3: Polyurethan-Dispersion 3

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht oder leicht unterschritten war. Das fertige Prepolymer wurde mit 4830 g Aceton gelöst und dabei auf 50°C abgekühlt und anschließend wurde eine Lösung aus 36,9 g 1,4-Diaminobutan, 116,5 g Isophorondiamin, 61,7 g Diaminosulfonat und 1076 g Wasser innerhalb von 10 min zudosiert. Die Nachrührzeit betrug 10 min. Dann wurde durch Zugabe von 1210 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 59% |
| Partikelgröße (LKS): | 350 nm |
| Viskosität (Viskosimeter, 23°C): | 126 mPas |
| pH (23°C): | 7,07 |

### Beispiel 4: Polyurethan-Dispersion 4

201,3 g PolyTHF^{®} 2000, 76,6 g PolyTHF^{®} 1000, 155,3 g Desmophen^{®} C2200, 2,50 g 1,4-Butandiol und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht oder leicht unterschritten war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 243 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 62% |
| Partikelgröße (LKS): | 566 nm |
| Viskosität (Viskosimeter, 23°C): | 57 mPas |
| pH (23°C): | 6,64 |

### Beispiel 5: Polyurethan-Dispersion 5

201,3 g PolyTHF^{®} 2000, 76,6 g PolyTHF^{®} 1000, 155,3 g Desmophen^{®} C2200, 2,50 g Trimethylolpropan und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht oder leicht unterschritten war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 293 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 56% |
| Partikelgröße (LKS): | 440 nm |
| Viskosität (Viskosimeter, 23°C): | 84 mPas |
| pH (23°C): | 6,91 |

### Beispiel 6: Polyurethan-Dispersion 6

1072 g PolyTHF^{®} 2000, 407,6 g PolyTHF^{®} 1000, 827 g Desmophen^{®} C2200 und 48,1 g Polyether LB 25 wurden in einer Standard-Rührapparatur auf 70°C aufgeheizt. Anschließend wurde bei 70°C innerhalb von 5 min ein Gemisch aus 257,4 g Hexamethylendiisocyanat und 340 g Isophorondiisocyanat zugegeben und bei 120°C gerührt, bis der theoretische NCO-Wert erreicht oder leicht unterschritten war. Das fertige Prepolymer wurde mit 4820 g Aceton gelöst und dabei auf 50°C abgekühlt und anschließend wurde eine Lösung aus 27,3 g Ethylendiamin, 126,5 g Isophorondiamin, 67,0 g Diaminosulfonat und 1090 g Wasser innerhalb von 10 min zudosiert. Die Nachrührzeit betrug 10 min. Dann wurde durch Zugabe von 1180 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 60% |
| Partikelgröße (LKS): | 312 nm |
| Viskosität (Viskosimeter, 23°C): | 286 mPas |
| pH (23°C): | 7,15 |

### Beispiele 7-12: Schäume, hergestellt aus den Polyurethan-Dispersionen der Beispiele 1-6

Die in der Tabelle 1 angegebenen Mengen der Polyurethan-Dispersionen, hergestellt wie in den Beispielen 1-6 beschrieben, wurden mit den Schaumhilfsmitteln wie dort ebenso angegeben vermischt und unter Verwendung eines handelsüblichen Handrührgerätes (Rührer aus gebogenem Draht) auf 1 Liter Schaumvolumen aufgeschlagen. Unter weiterem Rühren wurden die erhaltenen Schäume durch Zugabe von Praestol^{®} 185 K schließlich koaguliert; durch die Koagulation blieb das Schaumvolumen unverändert (leichte Viskositätszunahme). Danach wurden die Schäume mittels eines Filmziehgerätes (Rakel), Spalthöhe in der Tabelle 1 angegeben, auf siliconbeschichtetes Papier aufgezogen. In der Tabelle 1 sind ebenso die Trocknungsbedingungen der wie angegeben hergestellten Schäume aufgeführt. Es wurden durchweg reinweiße Schäume mit guten mechanischen Eigenschaften und einer feinen Porenstruktur erhalten.

**Tabelle 1**

| | Menge [g] | | | | | |
|---|---|---|---|---|---|---|
| Schaum Nr. | Polyurethan-Dispersion (Beispiel) | Stokal^{®} STA | Stokal^{®} SR | Praestol ^{®} 185 k | SH¹⁾ [mm] | Aushärtung |
| 1a | 235,0(1) | 4,2 | 5,6 | 5,0 | 2 | 2 h / 37°C |
| 1b | 235,0 (1) | 4,2 | 5,6 | 5,0 | 4 | 18 h / 37°C |
| 1c | 235,0 (2) | 4,2 | 5,6 | 5,0 | 6 | 18 h / 37°C |
| 1d | 235,0(2) | 4,2 | 5,6 | 5,0 | 4 | 18 h / 37°C, 30 min / 120°C |
| 1e | 235,0(2) | 4,2 | 5,6 | 5,0 | 6 | 18 h / 37°C, 30 min / 120°C |
| 2 | 235,0 (2) | 4,2 | 5,6 | 5,0 | 4 | 2 h / 37°C, 30 min / 120°C |
| 3 | 235,0 (3) | 4,2 | 5,6 | 5,0 | 4 | 18 h / 37°C |
| 4 | 235,0 (4) | 4,2 | 5,6 | 5,0 | 4 | 2 h / 37°C, 30 min / 120°C |
| 5 | 235,0 (5) | 4,2 | 5,6 | 5,0 | 4 | 2 h / 37°C, 30 min / 120°C |
| 6 | 235,0 (6) | 4,2 | 5,6 | 5,0 | 4 | 2 h / 37°C, 30 min / 120°C |

| | | | | | | |
|---|---|---|---|---|---|---|
| ¹⁾ Spalthöhe Rakel | | | | | | |

Wie der Tabelle 2 zu entnehmen ist, zeigten allen Schäume eine sehr schnelle Aufnahme von Wasser, eine hohe Absorption von physiologischer Salzlösung ("Saugleistung bei freier Quellmöglichkeit"), eine sehr gute Wasserdampfdurchlässigkeit ("MVTR") und darüber hinaus eine gute mechanische Festigkeit, insbesondere auch nach Feuchtelagerung.

**Tabelle 2**

| Schaum Nr. | Aufnahmegeschwindigkeit¹⁾ [s] | Freie Saugleistung²⁾ [g/100 cm²] | MVTR³⁾ [g/m²*24 h] |
|---|---|---|---|
| 1a | nicht bestimmt | 13,4 | 6500 |
| 1b | nicht bestimmt | 23,6 | 6300 |
| 1c | nicht bestimmt | 33,0 | 5100 |
| 1d | 9 | 20,1 | 4400 |
| 1e | 9 | 29,6 | 4200 |
| 2 | 7 | 21,4 | 4100 |
| 3 | 7 | 23,4 | 3700 |
| 4 | 18 | 20,2 | 4100 |
| 5 | 11 | 25,8 | 4300 |
| 6 | 17 | 22,1 | 4400 |

| | | | |
|---|---|---|---|
| 1) Zeit bis zum vollständigen Eindringen eines Tropfens destilliertes Wasser in den Schaum (Prüfung der zum Papier gerichteten Seite); ²⁾ Absorption physiologischer Salzlösung bestimmt nach DIN EN 13726-1, Teil 3.2 (5 anstelle von 9 Prüfmustern); ³⁾ "moisture vapour transition rate" (Wasserdampfdurchlässigkeit) bestimmt nach DIN EN 13726-2, Teil 3.2 | | | |

### Beispiel 13:

54 g einer nach Beispiel 2 hergestellten Polyurethan-Dispersion wurden mit 1,37 g Simulsol^{®} SL 26 vermischt. Diese Mischung wurde in eine Kammer einer geeigneten 2-Komponenten-Aerosol-Dose gegeben; die andere Kammer wurde mit 1,69 g Praestol^{®} 185 K gefüllt. Die Komponenten wurden schließlich mit 6 g einer Treibmittelmischung aus i-Butan/Propan/n-Butan versetzt. Nach dem Sprühen (ca. 1 cm Nassfilmdicke) und Trocknen bei Umgebungsbedingungen wurde ein reinweißer, feinzelliger Schaum erhalten.

## Patentansprüche

1. Verfahren zur Herstellung von Wundauflagen aus Polyurethan-Schäumen, bei dem eine Zusammensetzung enthaltend eine wässrige, anionisch hydrophilierte Polyurethan-Dispersionen (I) und ein kationisches Koagulans (II) aufgeschäumt und getrocknet wird.

2. Verfahren gemäß Anspruch 1, **dadurch gekennzeichnet, dass** die wässrigen, anionisch hydrophilierten Polyurethan-Dispersionen (I) erhältlich sind, indem
A) isocyanatfunktionelle Prepolymere aus
A1) organischen Polyisocyanaten
A2) polymeren Polyolen mit zahlenmittleren Molekulargewichten von 400 bis 8000 g/mol und OH-Funktionalitäten von 1,5 bis 6 und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und gegebenenfalls nichtionischen Hydrophilierungsmitteln,
hergestellt werden,
B) dessen freie NCO-Gruppen dann ganz oder teilweise
B1) gegebenenfalls mit aminofunktionellen Verbindungen mit Molekulargewichten von 32 bis 400 g/mol und
B2) aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

3. Verfahren gemäß Anspruch 2, **dadurch gekennzeichnet, dass** bei der Herstellung der wässrigen, anionisch hydrophilierten Polyurethan-Dispersionen (I) in A1) 1,6-Hexamethylendiisocyanat, Isophorondiisocyanat, die isomeren Bis-(4,4'-isocyanatocyclohexyl)methane, sowie deren Mischungen eingesetzt werden und in A2) eine Mischung aus Polycarbonatpolyolen und Polytetramethylenglykolpolyolen eingesetzt wird, wobei der Anteil der Summe der Polycarbonat- und Polytetramethylenglykolpolyetherpolyole an der Komponente A2) mindestens 70 Gew.-% beträgt.

4. Verfahren gemäß einem der Ansprüche 1 bis 3, **dadurch gekennzeichnet, dass** das kationische Koagulans (II) ein Copolymer des Acrylamids ist, welches Struktureinheiten der allgemeinen Formel (1) und (2) aufweist 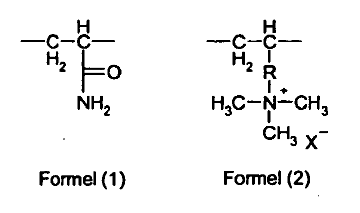 wobei
R C=O, -COO(CH₂)₂- oder -COO(CH₂)₃- und
X⁻ ein Halogenidion ist.

5. Verfahren gemäß einem der Ansprüche 1 bis 4, **dadurch gekennzeichnet, dass** neben der Polyurethan-Dispersion (I) und dem kationisches Koagulans (II) auch Hilfs- und Zusatzstoffe (III) enthalten sind.

6. Verfahren gemäß Anspruch 5, **dadurch gekennzeichnet, dass** als Hilfs- und Zusatzstoffe (III) wasserlösliche Fettsäureamide, Sulfosuccinamide, Kohlenwasserstoffsulfonate, -sulfate oder Fettsäuresalze als Schaumbildner und -stabilisatoren enthalten sind.

7. Verfahren gemäß Anspruch 6, **dadurch gekennzeichnet, dass** als Schaumbildner und -stabilisatoren Mischungen aus Sulfosuccinamiden und Ammoniumstearaten verwendet werden, wobei diese 70 bis 50 Gew.-% an Sulfosuccinamiden enthalten.

8. Wundauflagen erhältlich nach einem Verfahren gemäß einem der Ansprüche 1 bis 7.

9. Zusammensetzungen enthaltend eine wässrige, anionisch hydrophilierte Polyurethan-Dispersionen (I) und ein kationisches Koagulans (II), wobei das Koagulans (II) eine organische Verbindung umfasst, die mindestens zwei kationische Gruppen enthält.

## Claims

1. Process for producing wound contact materials made of polyurethane foams which comprises a composition containing an aqueous, anionically hydrophilicized polyurethane dispersion (I) and a cationic coagulant (II) being frothed and dried.

2. Process according to Claim 1, **characterized in that** the aqueous, anionically hydrophilicized polyurethane dispersions (I) are obtainable by
A) isocyanate-functional prepolymers being produced from
A1) organic polyisocyanates
A2) polymeric polyols having number-average molecular weights in the range from 400 to 8000 g/mol and OH functionalities in the range from 1.5 to 6 and
A3) optionally hydroxyl-functional compounds having molecular weights in the range from 62 to 399 g/mol and
A4) optionally isocyanate-reactive, anionic or potentially anionic and optionally nonionic hydrophilicizing agents
and
B) its free NCO groups then being wholly or partly reacted
B1) optionally with amino-functional compounds having molecular weights in the range from 32 to 400 g/mol and
B2) with amino-functional, anionic or potentially anionic hydrophilicizing agents
by chain extension, and the prepolymers being dispersed in water before, during or after step B), any potentially ionic groups present being converted into the ionic form by partial or complete reaction with a neutralizing agent.

3. Process according to Claim 2, **characterized in that** the aqueous, anionically hydrophilicized polyurethane dispersions (I) are produced using in A1) 1,6-hexamethylene diisocyanate, isophorone diisocyanate, the isomeric bis-(4,4'-isocyanatocyclohexyl)methanes and also mixtures thereof and in A2) a mixture of polycarbonate polyols and polytetramethylene glycol polyols, the proportion of component A2) which is contributed by the sum total of the polycarbonate and polytetramethylene glycol polyether polyols being at least 70% by weight.

4. Process according to any one of Claims 1 to 3, **characterized in that** the cationic coagulant (II) is an acrylamide copolymer comprising structural units of the general formula (1) and (2) 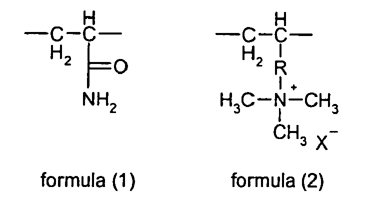 where
R is C=O, -COO(CH₂)₂- or -COO(CH₂)₃- and
X⁻ is a halide ion.

5. Process according to any one of Claims 1 to 4, **characterized in that** auxiliary and additive materials (III) are included as well as the polyurethane dispersion (I) and the cationic coagulant (II).

6. Process according to Claim 5, **characterized in that** as auxiliary and additive materials (III) there are included water-soluble fatty acid amides, sulfosuccinamides, hydrocarbyl sulfonates or sulfates, or fatty acid salts as foam formers and stabilizers.

7. Process according to Claim 6, **characterized in that** mixtures of sulfosuccinamides and ammonium stearates are used as foam formers and stabilizers, these mixtures containing 70% to 50% by weight of sulfosuccinamides.

8. Wound contact materials obtainable by a process according to any one of Claims 1 to 7.

9. Compositions containing an aqueous, anionically hydrophilicized polyurethane dispersion (I) and a cationic coagulant (II), wherein the coagulant (II) comprises an organic compound which contains at least two cationic groups.

## Revendications

1. Procédé pour la préparation de revêtements de plaies constitués de mousses de polyuréthane, dans lequel on fait mousser et on sèche une composition contenant une dispersion de polyuréthane aqueuse anioniquement hydrophilisée (I) et un agent de coagulation cationique (II).

2. Procédé selon la revendication 1, **caractérisé en ce que** les dispersions de polyuréthane aqueuses anioniquement hydrophilisées (I) sont obtenues **en ce que** l'on prépare
A) des prépolymères isocyanate-fonctionnels constitués
A1) de polyisocyanates organiques
A2) de polyols polymères avec des poids moléculaires moyens en nombre de 400 à 8 000 g/mol et des fonctionnalités OH de 1,5 à 6 et
A3) éventuellement des composés hydroxy-fonctionnels avec des poids moléculaires de 62 à 399 g/mol et
A4) des agents d'hydrophilisation éventuellement isocyanate-réactifs, anioniques ou potentiellement anioniques et éventuellement non ioniques,
B) dont les groupes NCO libres réagissent alors totalement ou partiellement
B1) éventuellement avec des composés amino-fonctionnels présentant des poids moléculaires de 32 à 400 g/mol et B2) avec des agents d'hydrophilisation amino-fonctionnels,
anioniques ou potentiellement anioniques
avec un allongement de chaîne et on disperse les prépolymères avant, pendant ou après l'étape B) dans de l'eau, dans lequel des groupes potentiellement ioniques éventuellement contenus sont transformés dans la forme ionique par réaction partielle ou totale avec un agent de neutralisation.

3. Procédé selon la revendication 2, **caractérisé en ce que** l'on utilise dans la préparation des dispersions de polyuréthane aqueuses, anioniquement hydrophilisées (I) dans A1) du diisocyanate de 1,6-hexa-méthylène, du diisocyanate d'isophorone, les bis-(4,4'-isocyanatocyclohexyl)méthane isomères ainsi que leurs mélanges et dans A2) un mélange de polycarbonatepolyols et de polytétraméthylèneglycolpolyols, la part de la somme des polycarbonates- et polytétraméthylèneglycolpolyétherpolyols par rapport au constituant A2) étant d'au moins 70 % en poids.

4. Procédé selon l'une quelconque des revendications 1 à 3, **caractérisé en ce que** l'agent de coagulation cationique (II) est un copolymère de l'acrylamide, lequel présente des unités de structure des formules générales (1) et (2) 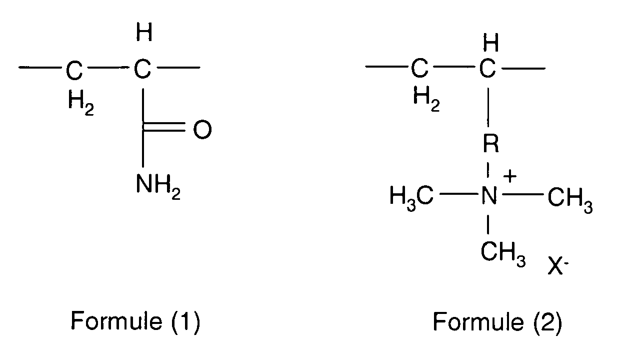 dans lesquelles
R est C=O, -COO(CH₂)₂- ou -COO(CH₂)₃- et
X⁻ est un ion halogénure.

5. Procédé selon l'une quelconque des revendications 1 à 4, **caractérisé en ce que** des auxiliaires et des additifs (III) sont également contenus en plus de la dispersion de polyuréthane (I) et de l'agent de coagulation cationique (II).

6. Procédé selon la revendication 5, **caractérisé en ce que** sont contenus comme auxiliaires et additifs (III) des amides d'acides gras, des sulfosuccinamides, des sulfonates d'hydrocarbures, des sulfates d'hydrocarbures ou des sels d'acides gras solubles dans l'eau en tant qu'agents de formation et stabilisateurs de mousse.

7. Procédé selon la revendication 6, **caractérisé en ce que** l'on utilise comme agents de formation et stabilisateurs de mousse des mélanges constitués de sulfosuccinamides et de stéarates d'ammonium, ceux-ci contenant de 70 à 50 % en poids de sulfosuccinamides.

8. Revêtements de plaies obtenus selon un procédé selon l'une quelconque des revendications 1 à 7.

9. Compositions contenant une dispersion aqueuse de polyuréthane anioniquement hydrophilisée (I) et un agent de coagulation cationique (II), l'agent de coagulation (II) contenant un composé organique qui présente au moins deux groupes cationiques.
